# EUROPEAN PATENT APPLICATION

(11) **EP 4 613 306 A1**
(43) Date of publication of application: **10.09.2025**
(21) Application number: 23886167.8
(22) Date of filing: 30.10.2023
(51) Int. Cl.: A61M 15/00

(54) **INHALER**

(30) Priority: 31.10.2022 KR 20220142444
(71) Applicant: KT&G Corporation, Daedeok-gu Daejeon 34337 (KR)
(72) Inventor: KIM, Moon Won, Daejeon 34128 (KR); JUNG, Yong Mi, Daejeon 34128 (KR); SHIN, Jun Won, Daejeon 34128 (KR); KI, Sung Jong, Daejeon 34128 (KR); JEOUNG, Eun Mi, Daejeon 34128 (KR); CHA, Sung Je, Daejeon 34128 (KR)
(74) Representative: Ter Meer Steinmeister & Partner
(86) International application number: PCT/KR2023/016974
(87) International publication number: WO 2024/096471

(57) **Abstract**

The inhaler according to an embodiment may comprise: an inhalation unit which allows a user to inhale a dry powder; and a storage unit which has the dry powder therein and may generate a cyclone-shaped airflow when the user inhales via the inhalation unit.

## Description

### TECHNICAL FIELD

The present disclosure relates to an inhaler.

### BACKGROUND ART

Recently, the demand for alternative articles to overcome the disadvantages of traditional cigarettes has increased. For example, an inhaler is an instrument used for a user to inhale a composition, such as a drug, as liquid or gas, through the oral or nasal cavity in an inhalation process. Such an inhaler may include a container accommodating an inhalable composition, and the composition may be sprayed from the container through a thin tube to the oral cavity or nasal cavity through an intake to be inhaled by a user.

As an example of the prior arts, European Patent Application No. 0017578 (March 19, 1980) discloses an inhaler.

The above description is information the inventor(s) acquired during the course of conceiving the present disclosure, or already possessed at the time, and was not necessarily publicly known before the present application was filed.

### DISCLOSURE OF THE INVENTION

### TECHNICAL GOALS

An object according to an embodiment is to provide an inhaler that uses a cyclone-shaped airflow.

An object according to an embodiment is to provide an inhaler that may improve the uniformity of an inhaled dry powder.

### TECHNICAL SOLUTIONS

An inhaler according to an embodiment includes an inhalation portion through which a user inhales a dry powder and a storage having the dry powder therein and configured to generate a cyclone-shaped airflow when the user inhales from the inhalation portion.

The storage may include at least one inlet through which air is introduced.

The inhalation portion may include an airflow channel through which air and the dry powder flow and a channel housing surrounding at least a portion of the airflow channel, in which a portion of the airflow channel may be inserted into the storage.

The storage and the airflow channel may be formed in a cylindrical shape.

A cross-sectional area of the portion of the airflow channel that is inserted into the storage may be formed narrower than an area of one end portion of the airflow channel.

The at least one inlet may be formed, on the storage, closer to the inhalation portion than one end portion of the airflow channel that is inserted into the storage.

The storage may include a cyclone chamber of which at least a portion is formed in a tapered shape so that the cyclone-shaped airflow is generated.

The inhaler may further include a cover portion configured to control opening and closing of the at least one inlet and the inhalation portion of the storage or both.

The cover portion may include a first cover configured to control the opening and closing of the at least one inlet of the storage and a second cover configured to control the opening and closing of the inhalation portion.

Each of the first cover and the second cover may include an adhesive element to be attached to the at least one inlet and the inhalation portion.

The inhaler may further include a support configured to support one end portion of the storage.

The support may include a porous material, a rigid material, or a combination thereof.

### EFFECTS OF THE INVENTION

An inhaler according to an embodiment may use a cyclone-shaped airflow.

An inhaler according to an embodiment may improve the uniformity of an inhaled dry powder.

### BRIEF DESCRIPTION OF DRAWINGS

FIG. 1 illustrates an inhaler according to an embodiment.
FIGS. 2A and 2B illustrate a cover portion of an inhaler according to an embodiment.
FIG. 3 illustrates a storage of an inhaler according to an embodiment.
FIG. 4 illustrates a storage of an inhaler according to an embodiment.
FIG. 5 illustrates a storage of an inhaler according to an embodiment.

### BEST MODE FOR CARRYING OUT THE INVENTION

Hereinafter, embodiments will be described in detail with reference to the accompanying drawings. The following description describes one of several aspects of the embodiments, and the following description forms part of the detailed description of the embodiments. In the description of an embodiment, any detailed description of a well-known function or configuration is not included to clearly convey the gist of the present disclosure.

However, various alterations and modifications may be made to the embodiments. Thus, the embodiments are not meant to be limited by the descriptions of the present disclosure. The embodiments should be understood to include all changes, equivalents, and replacements within the idea and the technical scope of the disclosure.

In addition, the terms or words used to describe the present disclosure and claims should not be construed in a conventional or dictionary meaning, and based on a principle that the inventor may properly define the concept of terms in order to best describe their invention, the terms or words should be construed as having meanings and concepts consistent with the technical idea of the disclosure according to an embodiment.

The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. Terms defined in dictionaries generally used should be construed as having meanings matching contextual meanings in the related art and are not to be construed as having an ideal or excessively formal meaning unless otherwise defined herein.

When describing the embodiments with reference to the accompanying drawings, like reference numerals refer to like constituent elements and a repeated description related thereto will be omitted. In the description of embodiments, detailed description of well-known related technology will be omitted when it is deemed that such description will cause ambiguous interpretation of the present disclosure.

Also, in the description of the components of the embodiments, terms such as first, second, A, B, (a), (b), and the like may be used. These terms are used only for the purpose of discriminating one component from another component, and the nature, the sequences, or the orders of the components are not limited by the terms. It is to be understood that if a component is described as being "connected," "coupled" or "joined" to another component, the former may be directly "connected," "coupled," and "joined" to the latter or "connected", "coupled", and "joined" to the latter via another component.

Components included in one embodiment and components that include common functions will be described using the same name in other embodiments. Unless otherwise mentioned, the descriptions of the embodiments may be applicable to the following embodiments and thus, duplicated descriptions will be omitted for conciseness.

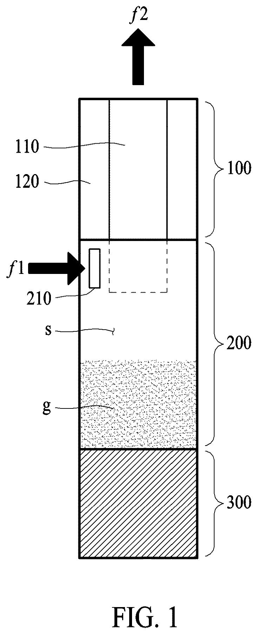

FIG. 1 illustrates an inhaler according to an embodiment.

Referring to FIG. 1, the inhaler according to an embodiment may include an inhalation portion 100, a storage 200, and a support 300.

A user may inhale from the inhaler by applying negative pressure to the inhalation portion 100. The inhalation portion 100 may include a mouthpiece shape from which a user may make an inhalation. The inhalation portion 100 may include an airflow channel 110 through which an airflow may flow inward and a channel housing 120 structurally surrounding the airflow channel 110. A portion of the airflow channel 110 may be inserted into the storage 200.

The storage 200 may be connected to the inhalation portion 100. The airflow channel 110 of the inhalation portion 100 may penetrate one end portion of the storage 200. An inhalable dry powder g may be provided inside the storage 200. The dry powder g provided inside the storage 200 may include a functional material, such as caffeine, taurine, or the like, a pharmacological material, such as aspirin, a sedative, a sleeping pill, a bronchodilator, a vaccine, or the like, or a material, such as a nicotine-free material, nicotine salts, or the like. The storage 200 may include a cavity s to contain the dry powder g. When the user inhales from the inhaler by applying negative pressure to the inhalation portion 100, the airflow channel 110 of the inhalation portion 100 may suck air inside the storage 200 and form negative pressure inside the storage 200. The storage 200 may include an inlet 210 for introducing external air. When the user inhales from the inhalation portion 100, negative pressure may be formed inside the storage 200 and air may be introduced into the inlet 210 formed on the storage 200. Air f1 introduced into the storage 200 may be inhaled f2 by the user through the airflow channel 110 that is inserted back into the storage 200.

The support 300 may be disposed to touch the storage 200. The support 300 may structurally support the storage. The support 300 may form the exterior of the inhaler together with the inhalation portion 100 and the storage 200. The support 300 may include a porous medium or a rigid material. The support 300 may include a porous medium such as acetate tow, etc.

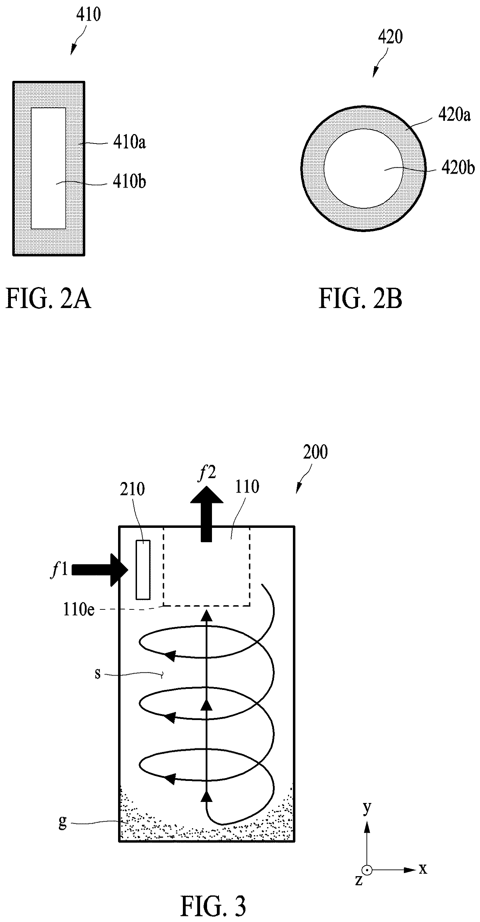

FIGS. 2A and 2B illustrate a cover portion of an inhaler according to an embodiment.

The cover portion may open or close the inlet 210 formed on the storage 200, the airflow channel 110 of the inhalation portion 100 through which the user inhales, or both. The storage 200 may include a first cover and a second cover.

More specifically, FIG. 2A illustrates the first cover of the inhaler according to an embodiment. A first cover 410 may open or close an inlet formed on a storage. The first cover 410 may be formed in a shape corresponding to the shape of the inlet of the storage. For example, when the inlet of the storage is formed in the shape of a slit, the first cover 410 may have the shape of a sealing member formed long in a longitudinal direction of the slit. The first cover 410 may include an adhesive element 410a formed to be attached to the storage. On the first cover 410, an area 410b, excluding the adhesive element 410a, may shield the inlet of the storage. On the first cover 410, the inlet is shielded by the area 410b, excluding the adhesive element 410a, so it may be possible to prevent a dry powder inside the storage from adhering to the adhesive element 410a. On the first cover 410, the area 410b, excluding the adhesive element 410a, may include a coating layer to prevent the dry powder inside the storage from adhering. The first cover 410 may be repeatedly attached or detached to or from the storage. The shape of the first cover 410 and the adhesive element 410a may vary depending on the shape of the inlet formed on the storage and is not limited to the shape illustrated in FIG. 2A.

FIG. 2B illustrates the second cover of the inhaler according to an embodiment. A second cover 420 may open or close an airflow channel of an inhalation portion. The second cover 420 may be formed in a shape corresponding to the cross-section shape of the airflow channel of the inhalation portion. For example, when the airflow channel of the inhalation portion is formed in a cylindrical shape, the second cover 420 may have a shape of a circular sealing member. The second cover 420 may include an adhesive element 420a formed to be attached to a channel housing of the inhalation portion. On the second cover 420, an area 420b, excluding the adhesive element 420a, may shield the airflow channel of the inhalation portion. On the second cover 420, the airflow channel is shielded by the area 420b, excluding the adhesive element 420a, so it may be possible to prevent the dry powder inside the airflow channel from adhering to the adhesive element 420a. On the second cover 420, the area 420b, excluding the adhesive element 420a, may include a coating layer to prevent the dry powder remaining inside the airflow channel from adhering. The second cover 420 may be repeatedly attached or detached to or from the inhalation portion. The shape of the second cover 420 and the adhesive element 420a may vary depending on the shape of the airflow channel and the channel housing and is not limited to the shape illustrated in FIG. 2B.

FIG. 3 illustrates a storage of an inhaler according to an embodiment.

Referring to FIG. 3, when a user inhales from the inhaler, a cyclone-shaped airflow may be generated inside the storage 200. When using the cyclone-shaped airflow, the flow may be separated according to the sizes of particles flowing internally by using a circling flow of a fluid.

When the user inhales from an inhalation portion, external air may be introduced f1 through the inlet 210 of the storage 200. Here, a portion of the inlet 210 may be formed to be closer to the inhalation portion than an end portion 110e of the airflow channel 110 that is inserted into the storage 200. External air introduced through the inlet 210 may form a cyclone-shaped airflow by circling along an inner side surface of the storage 200, and the formed cyclone-shaped airflow may flow f2 toward the inhalation portion along the airflow channel by flowing together with the dry powder g provided inside the storage 200. As described below, an inner space s of the storage 200 may be designed to have a preset shape so that the cyclone-shaped airflow is easily generated. Air introduced f1 through the inlet 210 may descend (e.g., in a -y direction) while circling along a tangential direction of the inner side surface of the storage 200 and may rise again in a direction (e.g., a +y direction) toward the airflow channel together with the dry powder g provided inside the storage. In this process, depending on the diameter of the storage 200, the particles of the dry powder g, which have sizes less than or equal to a preset size, may flow in a direction of the airflow channel and be inhaled f1 by the user through the inhalation portion, and the particles having sizes greater than or equal to the preset size may remain inside the storage 200 without deviating from the cyclone airflow that flows while circling inside the storage 200. The storage 200 may desirably be formed in a cylindrical shape.

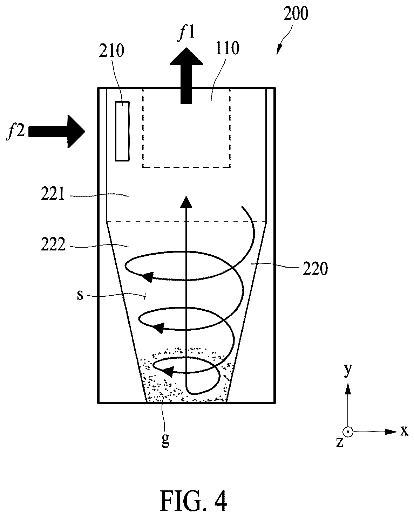

FIG. 4 illustrates a storage of an inhaler according to an embodiment.

Referring to FIG. 4, when a user inhales from an inhalation portion, external air may be introduced f2 through the inlet 210 of the storage 200, and a cyclone-shaped airflow may be generated inside the storage 200. Here, a portion of the inlet 210 may be formed to be closer to the inhalation portion than an end portion of the airflow channel 110 that is inserted into the storage 200. External air introduced through the inlet 210 may form the cyclone-shaped airflow by circling along the inner side surface of the storage 200, and the formed cyclone-shaped airflow may flow f1 toward the inhalation portion along the airflow channel by flowing together with the dry powder g provided inside the storage 200.

Here, the storage 200 may include a cyclone chamber 220 of which at least a portion is formed in a tapered shape so that the cyclone-shaped airflow may be more easily generated in the inner space s of the storage 200. The cyclone chamber 220 provided inside the storage 200 may include a first chamber part 221 in which the inserted airflow channel 110 may be disposed and a second chamber part 222 extending from the first chamber part 221 and having a gradually decreasing cross-sectional area.

The inlet 210 may be formed on the first chamber part 221, and air introduced f2 through the inlet 210 may descend (e.g., in a -y direction) while circling along a tangential direction of an inner side surface of the second chamber part 222. Here, due to the shape of the second chamber part 222, it may be easier for the cyclone-shaped airflow to be generated. The cyclone-shaped airflow generated in the second chamber part 222 may rise again in a direction (e.g., a +y direction) toward the airflow channel together with the dry powder g provided internally. In this process, depending on the diameter of the storage 200, the particles of the dry powder g, which have sizes less than or equal to a preset size, may flow f1 in a direction of the airflow channel and be inhaled by the user through the inhalation portion, and the particles having sizes greater than or equal to the preset size may remain inside the storage 200 without deviating from the cyclone airflow that flows while circling inside the storage 200.

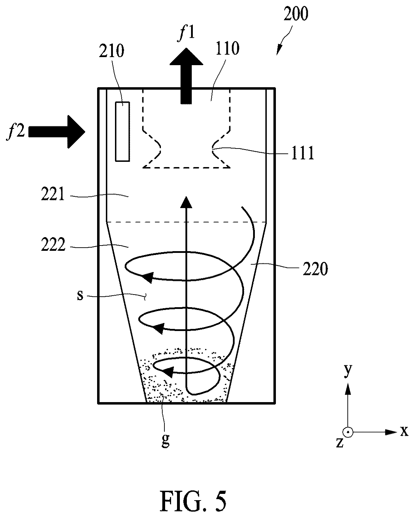

FIG. 5 illustrates a storage of an inhaler according to an embodiment.

Referring to FIG. 5, when a user inhales from an inhalation portion, external air may be introduced f2 through the inlet 210 of the storage 200, and a cyclone-shaped airflow may be generated inside the storage 200. Here, a portion of the inlet 210 may be formed to be closer to the inhalation portion than the end portion of the airflow channel 110 that is inserted into the storage 200. External air introduced through the inlet 210 may form the cyclone-shaped airflow by circling along the inner side surface of the storage 200, and the formed cyclone-shaped airflow may flow f1 toward the inhalation portion along the airflow channel by flowing together with the dry powder g provided inside the storage 200.

Here, the storage 200 may include the cyclone chamber 220 of which at least a portion is formed in a tapered shape so that the cyclone-shaped airflow may be more easily generated in the inner space s of the storage 200. The cyclone chamber 220 provided inside the storage 200 may include the first chamber part 221 in which the inserted airflow channel 110 may be disposed and the second chamber part 222 extending from the first chamber part 221 and having a gradually decreasing cross-sectional area.

The inlet 210 may be formed on the first chamber part 221, and air introduced f2 through the inlet 210 may descend (e.g., in a -y direction) while circling along the tangential direction of the inner side surface of the second chamber part 222. Here, due to the shape of the second chamber part 222, it may be easier for the cyclone-shaped airflow to be generated. The cyclone-shaped airflow generated in the second chamber part 222 may rise again in a direction (e.g., a +y direction) toward the airflow channel together with the dry powder g provided internally. In this process, depending on the diameter of the storage 200, the particles of the dry powder g, which have sizes less than or equal to a preset size, may flow f1 in a direction of the airflow channel and be inhaled by the user through the inhalation portion, and the particles having sizes greater than or equal to the preset size may remain inside the storage 200 without deviating from the cyclone airflow that flows while circling inside the storage 200.

Here, the cross-sectional area of a portion of the airflow channel 110 that is inserted into the storage 200 may be formed narrower than an area of one end portion of the airflow channel. The airflow channel 110 inserted into the storage 200 may be formed to include a shape of a channel neck 111 of which a cross-sectional area gradually decreases and then increases. The channel neck 111 of the airflow channel 110 may prevent a large amount of dry powder g from being inhaled at a time and may obtain a more uniform inhalation feeling by controlling the amount of airflow moving to the inhalation portion.

Embodiments have been described above with reference to specific matters such as specific components and limited embodiments and with reference to drawings, but these are provided to facilitate overall understanding. Also, the present disclosure is not limited to the above-described embodiments, and various modifications and variations are possible from these descriptions by those skilled in the art to which the present disclosure pertains. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
an inhalation portion through which a user inhales a dry powder;
a storage having the dry powder therein and configured to generate a cyclone-shaped airflow when the user inhales from the inhalation portion.

2. The inhaler of claim 1, wherein the storage comprises at least one inlet through which air is introduced.

3. The inhaler of claim 2, wherein the inhalation portion comprises an airflow channel through which air and the dry powder flow and a channel housing surrounding at least a portion of the airflow channel,
wherein a portion of the airflow channel is inserted into the storage.

4. The inhaler of claim 3, wherein the storage and the airflow channel are formed in a cylindrical shape.

5. The inhaler of claim 3, wherein a cross-sectional area of the portion of the airflow channel that is inserted into the storage is formed narrower than an area of one end portion of the airflow channel.

6. The inhaler of claim 3, wherein the at least one inlet is formed, on the storage, closer to the inhalation portion than one end portion of the airflow channel that is inserted into the storage.

7. The inhaler of claim 1, wherein the storage comprises a cyclone chamber of which at least a portion is formed in a tapered shape so that the cyclone-shaped airflow is generated.

8. The inhaler of claim 2, further comprising:
a cover portion configured to control opening and closing of the at least one inlet and the inhalation portion of the storage or both.

9. The inhaler of claim 8, wherein the cover portion comprises a first cover configured to control the opening and closing of the at least one inlet of the storage and a second cover configured to control the opening and closing of the inhalation portion.

10. The inhaler of claim 9, wherein each of the first cover and the second cover comprises an adhesive element to be attached to the at least one inlet and the inhalation portion.

11. The inhaler of claim 1, further comprising:
a support configured to support one end portion of the storage.

12. The inhaler of claim 11, wherein the support comprises a porous material, a rigid material, or a combination thereof.
